# EUROPEAN PATENT SPECIFICATION

(11) **EP 4 180 814 B1**
(45) Date of publication and mention of the grant of the patent: **04.03.2026**
(21) Application number: 23150361.6
(22) Date of filing: 30.03.2017
(51) Int. Cl.: G01N 33/574

(54) **MARKERS OF ENDOMETRIAL CANCER**
MARKER FÜR ENDOMETRIUMKREBS
MARQUEURS DU CANCER DE L'ENDOMÈTRE

(30) Priority: 04.05.2016 EP 16168328
(43) Date of publication of application: 17.05.2023
(62) Divisional of application: 17713973.0
(73) Proprietor: Fundació Hospital Universitari Vall d'Hebron - Institut de Recerca, 08035 Barcelona (ES)
(72) Inventor: MARTINEZ GARCÍA, Elena, 09002 Burgos (ES); COLAS ORTEGA, Eva, 08020 Barcelona (ES); GIL MORENO, Antonio, 08172 Sant Cugat del Vallès (ES); REVENTÓS PUIGJANER, Jaume, 08348 Cabrils (ES); DOMON, Bruno, 2534 Orvin (CH); LESUR, Antoine, 8010 Strassen (LU)
(74) Representative: Hoffmann Eitle

(56) References cited:
- US-B1- 6 607 894
- ALEX LOPATA ET AL: "Detection of endometrial cancer by determination of matrix metalloproteinases in the uterine cavity", GYNECOLOGIC ONCOLOGY., vol. 90, no. 2, 1 August 2003 (2003-08-01), GB, pages 318 - 324, XP055368824, ISSN: 0090-8258, DOI: 10.1016/S0090-8258(03)00328-7
- S. M. LAIRD ET AL: "Metalloproteinases and tissue inhibitor of metalloproteinase 1 (TIMP-1) in endometrial flushings from pre- and post-menopausal women and from women with endometrial adenocarcinoma", REPRODUCTION, vol. 115, no. 2, 1 March 1999 (1999-03-01), GB, pages 225 - 232, XP055368813, ISSN: 1470-1626, DOI: 10.1530/jrf.0.1150225
- LOMNYTSKA M I ET AL: "Impact of genomic stability on protein expression in endometrioid endometrial cancer", vol. 106, no. 7, 1 March 2012 (2012-03-01), London, pages 1297 - 1305, XP093028687, ISSN: 0007-0920, Retrieved from the Internet <URL:https://www.nature.com/articles/bjc201267.pdf> DOI: 10.1038/bjc.2012.67
- LOIS A. SALAMONSEN ET AL: "Proteomics of the human endometrium and uterine fluid: a pathway to biomarker discovery", FERTILITY AND STERILITY, vol. 99, no. 4, 1 March 2013 (2013-03-01), NL, pages 1086 - 1092, XP055495380, ISSN: 0015-0282, DOI: 10.1016/j.fertnstert.2012.09.013

## Description

This application claims the benefit of European Patent Application EP16168328.9 filed May 4th, 2016.

The invention relates to the diagnosis and prognosis of endometrial carcinoma.

### BACKGROUND ART

Endometrial cancer (EC) is the most frequently observed invasive tumor of the female genital tract and the fourth most common cancer in women in developed countries, accounting for 54,870 diagnosed cases and 10,170 estimated deaths in 2015 in the United States. Nowadays, 70% of the EC cases are diagnosed at early stages of the disease where the tumor is still localized within the endometrium and is associated with an overall 5-year survival rate of 96%. However, 30% of EC patients are diagnosed only at an advanced stage of the disease associated with a drastic decrease in the 5-year survival rate, which is reduced to 67% when myometrial invasion and/or lymph node affectation is already present and to 18% in cases of distant metastasis. Improving early diagnosis is hence a major issue to appropriately manage EC and decrease mortality associated with the disease.

Early detection of EC patients is favored by the presence of symptoms like abnormal vaginal bleeding present in 93% of women diagnosed with EC. However, many other benign disorders generate similar symptoms. Discrimination of patients with benign endometrial pathologies and with EC is only achieved after a tedious diagnostic process consisting of a pelvic examination and transvaginal ultrasonography followed by a confirmatory histopathological examination of an endometrial biopsy. The preferable biopsy used in this procedure is named uterine aspirate and/or pipelle biopsy and is obtained by a minimally invasive aspiration of endometrial fluid from inside the uterine cavity. Because the current diagnostic procedures on uterine aspirates rely on the presence of cellular material, this process has unfortunately a diagnostic failure and an associated inadequate sampling rate of 8% and 15%, respectively. This is increased in postmenopausal women up to 12% and 22%. In those cases, a biopsy guided by hysteroscopy needs to be performed, where this invasive technique presents an increased risk of complications, including uterine perforation, hemorrhage and possible harm to other organs.

Up to now, the development of proteomic-based diagnostic assays remained challenging, in spite of the many studies on EC tumor tissues and normal endometrium. The absence of translation of the results produced by those studies in the clinic is explained by two determinant factors: i) lack of studies in biofluids to achieve EC biomarkers. Most of the studies were based in tissues, and/or serum or plasma. However, the search of biomarkers in plasma or serum is extremely challenging due to the low concentration of the potential biomarkers and the wide dynamic range in protein abundance; and ii) lack of verification studies as a bridge between discovery and validation phases of the biomarker pipeline. Biomarker discovery experiments are fraught with false discoveries resulting from biological variability and small number of samples included.

Therefore, in spite of the efforts made, there is still the need of biomarkers allowing the diagnosis of endometrial cancer in early stages with high sensitivity and specificity.

### SUMMARY OF THE INVENTION

The present inventors have found that uterine fluid samples comprise several robust markers which make them appropriate samples for the diagnosis of endometrial cancer with high sensitivity and specificity.

As it is shown below, the present inventors have been able of identifying, for the first time, 27 proteins which are differentially expressed in uterine fluid samples from patients suffering endometrial cancer. Surprisingly, all 27 proteins show very high sensitivity and specificity (see Table 1 below), thus minimizing the risk of false positive or negative diagnosis.

These findings open the door to the use of uterine fluid samples as biological samples for the diagnosis of the disease instead of blood/serum or tissue biopsy. A useful diagnostic biomarker not only has to ameliorate the discrimination between patients suffering the disease and benign cases, but also should be economically profitable and advantageous in clinical scenario. In the case of diagnostic biomarkers for EC, reduction of number of invasive biopsies and diagnostic costs are very important values. Therefore, the identification of the biomarkers, object of the present invention, in an easy-to-access biofluid such as a uterine fluid sample, which is obtained in a minimally invasive procedure already implemented in the current diagnostic process, means a noticeable advance in the early diagnosis of the disease.

Therefore, the present invention means a great advance in the early diagnosis of endometrial cancer.

Thus, in a first aspect the present invention provides a method of diagnosis or prognosis endometrial carcinoma, the method comprising determining the level of expression of CLIC1; in an isolated fluid sample from the female genital tract.

It is remarkable that the biomarkers of the first aspect of the invention were individually and collectively associated with cancer, concluding that they maintained a strong association with commonly altered molecular processes in cancer such as cellular movement, cellular death and survival, among others.

Therefore, in a second aspect, the present invention provides the use of CLIC1 as an in vitro marker for diagnosing or prognosing endometrial carcinoma in an isolated fluid from the female genital tract. This aspect can also be formulated as a method for detecting one or more endometrial cancer markers in a subject, comprising: (a) obtaining a fluid sample from the female genital tract; and (b) detecting in the sample an amount of the endometrial cancer marker CLIC1.

In a third aspect, the present invention provides the use of means for determining the level of expression of CLIC1, for diagnosing or prognosing endometrial carcinoma in the method of the first aspect of the invention.

Importantly, the protein biomarkers object of the present invention can be assessed by easy and low-cost methods, such as immunochemistry or ELISA, platforms which are widely available in hospitals. Consequently, these protein biomarkers can be easily implemented as routine clinical diagnostic kits with reduced costs for the health system. In addition, a diagnostic kit test based on the biomarkers provided by the present invention can ameliorate the current process of diagnosis, conferring to uterine aspirates the ability of providing valuable diagnostic or prognostic information of the disease.

In a further aspect, the present invention provides a method for identifying a subject suspicious of suffering from endometrial carcinoma, the method comprising:
- a) determining, in vitro, the level of expression of the protein CLIC1in a fluid sample from the subject's female genital tract, and
- b) comparing the level of step (a) with a reference control level, wherein if the level determined in step (a) is higher than the reference control level, it is indicative that the subject is suspicious of suffering endometrial carcinoma.

In a further aspect, the present invention provides a method of deciding or recommending whether to initiate a medical regimen of a subject suspicious of suffering endometrial carcinoma, which method comprises the steps of:
- a) determining, in vitro, the level of expression of the protein CLIC1; and
- b) diagnosing the endometrial carcinoma or determining whether the subject is suspicious of suffering endometrial carcinoma, if the protein level in the test sample is higher than a reference control level;
wherein:
- i) if the subject is diagnosed of suffering from endometrial carcinoma, or of being suspicious of suffering from endometrial carcinoma, then the initiation of the medical regimen is recommended, and
- ii) if the patient is diagnosed of not suffering from endometrial carcinoma, the follow-up is performed optionally in consideration of the result of an examination of the patient by a physician.

By determining the marker level in a test sample, the skilled person can establish, additionally, which is the most suitable therapy that can be recommended, because the level detected in the sample may reflect the extension (i.e., severity) of the disease.

Furthermore, when it is decided that a subject has to initiate a medical regimen because she suffers from, or is suspicious of having, endometrial carcinoma, it can be monitored how efficient is the regimen using the markers of the invention: a decrease or return to a normal level of the marker (i.e., to the level of a cancer-free control subject) can indicate that the patient has reacted favourably to the medical regimen and, therefore, said regimen is effective; if the level of the marker does not significantly change or it increases, this can indicate that the regimen is not effective. Finally, the level of the marker can be measured after the end of the treatment for controlling relapses.

Therefore, in a further aspect, the present invention provides a method for determining the efficacy of a medical regimen in a patient already diagnosed of endometrial carcinoma, the method comprising the steps of:
- (a) in vitro measuring the level of expression of the protein CLIC1 in a fluid sample from the subject's female genital tract prior to the administration of the medical regimen;
- (b) in vitro measuring the level of said marker(s) in a fluid sample from the subject's female genital tract once started the administration of the medical regimen; and
- (c) comparing the levels measured in steps (a) and (b), in such a way that if the level measured in step (b) is lower than the level measured in step (a), it is indicative that the medical regimen is effective in the treatment of endometrial carcinoma;
or, alternatively, the method comprising the steps of:
- (i) in vitro measuring the level of expression of the protein CLIC1 in a fluid sample from the subject's female genital tract once started the administration of the medical regimen; and
- (ii) comparing the level measured in step (i) with a reference control level of the marker(s),
wherein, if the level measured in step (i) is not higher than the reference control level, it is indicative that the medical regimen is effective in the treatment of endometrial carcinoma.

With this method of the of the invention, it can be determined the treatment outcome (evaluation undertaken to assess the results or consequences of management and procedures used in combating disease in order to determine the efficacy, effectiveness, safety, practicability, etc., of these interventions in individual cases or series).

In a final aspect, the invention provides a workflow for the verification of potential protein markers of endometrial cancer by conducting a mass spectrometry analysis in targeted acquisition mode. From a list of potential endometrial cancer protein markers, at least one surrogate peptide per protein was selected according to criteria of detectability by mass spectrometry and uniqueness of the amino acids sequences. A version of those peptides that includes an amino acid labeled with stable heavy isotopes of carbon and nitrogen was synthetized. The fluid from the female genital tract were individually proteolyzed by trypsin and supplemented by an equal amount of the stable isotope labeled synthetic peptides mixture. Samples were analyzed by high performance liquid chromatography hyphenated with a hybrid high resolution mass spectrometer by: (a) generating an acquisition method that includes the list of peptide ions to be detected associated with their elution times under defined chromatographic conditions of separation; (b) conducting the mass spectrometry analysis by repeated isolation during elution time windows of the listed peptide ions by a quadrupole analyzer; (c) by performing a collision induced fragmentation of the isolated peptide ions; (d) and by analyzing of the subsequent peptides fragment ions with a high resolution analyzer. For each peptide, the signal of the fragment ions of interest was extracted to build elution profiles of peptides that can be integrated. The normalization of the areas resulting from endogenous peptides was performed with the area of the respective stable isotope labeled peptides. The method includes a step to confirm peptides identities by spectral matching.

### DETAILED DESCRIPTION OF THE INVENTION

All terms as used herein in this application, unless otherwise stated, shall be understood in their ordinary meaning as known in the art. Other more specific definitions for certain terms as used in the present application are as set forth below and are intended to apply uniformly through-out the specification and claims unless an otherwise expressly set out definition provides a broader definition.

The present invention provides new biomarkers for the diagnosis or prognosis of endometrial carcinoma in the female genital tract fluid.

The term "diagnosis" is known to the person skilled in the art. As used herein "diagnosis" is understood as becoming aware of a particular medical condition complication or risk in a subject; the determination of the nature of the disease or condition; or the distinguishing of one disease or condition from another. It refers both to the process of attempting to determine or identify the possible disease or disorder, and to the opinion reached by this process. A diagnosis, in the sense of diagnostic procedure, can be regarded as an attempt at classification of an individual's condition into separate and distinct categories that allow medical decisions about treatment and prognosis to be made. Subsequently, a diagnostic opinion is often described in terms of a disease or other condition. However, a diagnosis can take many forms. It might be a matter of detecting the presence and naming the disease, lesion, dysfunction or disability. It might be an exercise to attribute a category for management or for prognosis. It may indicate either degree of abnormality on a continuum or kind of abnormality in a classification.

The in vitro diagnostic method of the first aspect of the invention can be performed with a sample of: (a) an asymptomatic subject, (b) a subject which has already been identified as being suspicious of suffering from endometrial carcinoma, (c) a subject already diagnosed of endometrial carcinoma, as complementary confirmation diagnostic assay or (d) a subject with high risk of suffering the disease.

"Prognosis" as used herein refers to the prediction of the probable progression and outcome of a disease. It includes: neoplasm grading (attempt to express in replicable terms the level of cell differentiation in neoplasms as increasing anaplasia correlates with the aggressiveness of the neoplasm), neoplasm staging (attempt to express in replicable terms the extent of the neoplasm in the patient).

The term "fluid sample from the female genital tract" refers to a fluid produced by the uterine organ forming part of the female genital tract and which has been taken by aspiration, such as vacuum aspiration (i.e., "aspirate sample"). According to the present invention, the aspiration of the fluid is performed without a previous step of saline infusion. That is, the term "aspirate" does not encompass those samples resulting from uterine washings.

In another embodiment of the method of the first aspect of the invention, optionally in combination with any of the embodiments provided above or below, the method comprises (a) measuring, in vitro, the level of expression the protein CLIC1, in the test sample; and (b) compared the level of expression of each one of the tested proteins with a reference control value. In another embodiment of the method of the first aspect of the invention, optionally in combination with any of the embodiments provided above or below, the method comprises (a) measuring, in vitro, the level of expression of the protein CLIC1, in the test sample; and (b) compared the level of expression of each one of the tested proteins with a reference control value, wherein if proteins are overexpressed, it is indicative of endometrial carcinoma or bad prognosis.

In the present invention, the term "reference control level" referred to in the methods of the first and second aspects of the invention is to be understood as a predefined value of a given molecular marker, in the present case any of the proteins listed in the first or second aspects as well as in particular embodiments, which is derived from the levels of said molecular marker in a sample or group of samples. If the level of expression is determined at the protein level, then the "reference expression level" is a predefined value of protein quantity, whereas if the level of expression is determined at the mRNA level, then the "reference expression level" is a predefined value of mRNA quantity. The samples are taken from a subject or group of subjects wherein the presence, absence, stage, or course of the disease has been properly performed previously. This value is used as a threshold to discriminate subjects wherein the condition to be analyzed is present from those wherein such condition is absent (i.e. subject having endometrial cancer from subjects free of endometrial cancer), to determine the stage of the disease, the risk of developing or of being suffering from endometrial carcinoma, among others. This reference control level is also useful for determining whether the subject has to initiate a medical regimen and how effective the regimen is. The subject or subjects from whom the "reference control level" is derived may include subject/s wherein the condition is absent, subject/s wherein the condition is present, or both. The skilled person in the art, making use of the general knowledge, is able to choose the subject or group of subjects more adequate for obtaining the reference control level for each of the methods of the present invention. Methods for obtaining the reference value from the group of subjects selected are well-known in the state of the art (Burtis C. A. et al., 2008, Chapter 14, section "Statistical Treatment of Reference Values ") In a particular case "reference control level" is a cut-off value defined by means of a conventional ROC analysis (Receiver Operating Characteristic analysis). As the skilled person will appreciate, optimal cut-off value will be defined according to the particular applications of the diagnostic or prognostic method: purpose, target population for the diagnosis or prognosis, balance between specificity and sensibility, etc.

In another embodiment of the first aspect of the invention, optionally in combination with any of the embodiments provided above or below, the method further comprises determining the level of expression of one or more proteins selected from the group consisting of: ENOA, KPYM, PDIA1, ANXA2 and FABP5.

PERM, also known as myeloperoxidase or MPO, has the Uniprot database accession number P05164, February 19, 2014 - v4. MPO is a protein released by leukocytes that plays a crucial role in inflammation and oxidative stress in the cellular level.

CADH1, also known as cadherin-1 or E-cadherin, has the Uniprot database accession number P12830, July 1, 1993 - v3. This protein is involved in mechanisms regulating cell-cell adhesions, mobility and proliferation of epithelial cells. Has a potent invasive suppressor role.

SPIT1, also known as Kunitz-type protease inhibitor 1, has the Uniprot database accession number 043278, March 15, 2005 - v2. This protein is an inhibitor of HGF activator. Also acts as an inhibitor of matriptase (ST14).

ENOA, also known as alpha-enolase, has the Uniprot database accession number P06733, January 23, 2007 - v2. It is a multifunctional enzyme that, as well as its role in glycolysis, plays a part in various processes such as growth control, hypoxia tolerance and allergic responses. May also function in the intravascular and pericellular fibrinolytic system due to its ability to serve as a receptor and activator of plasminogen on the cell surface of several cell-types such as leukocytes and neurons. Stimulates immunoglobulin production.

MMP9, also known as matrix metalloproteinase-9, has the Uniprot accession number P14780, November 24, 2009 - v3. This protein may play an essential role in local proteolysis of the extracellular matrix and in leukocyte migration. Could play a role in bone osteoclastic resorption. Cleaves KiSS1 at a Gly-|-Leu bond. Cleaves type IV and type V collagen into large C-terminal three quarter fragments and shorter N-terminal one quarter fragments. Degrades fibronectin but not laminin or Pz-peptide
NAMPT, also known as nicotinamide phosphoribosyltransferase, has the Uniprot database accession number P43490, November 1, 1995 - v1. This enzyme, which is the rate limiting component in the mammalian NAD biosynthesis pathway, catalyzes the condensation of nicotinamide with 5-phosphoribosyl-1-pyrophosphate to yield nicotinamide mononucleotide, an intermediate in the biosynthesis of NAD.

LDHA, also known as L-lactate dehydrogenase A chain, has the Uniprot database accession number P00338, January 23, 2007 - v2. This protein is involved in step 1 of the subpathway that synthesizes (S)-lactate from pyruvate.

CASP3, also known as caspase-3, has the Uniprot database accession number P42574, October 11, 2005 - v2. It is involved in the activation cascade of caspases responsible for apoptosis execution.

KPYM, also known as pyruvate kinase PKM, has the Uniprot database accession number P14618, January 23, 2007 - v4. It is a glycolytic enzyme that catalyzes the transfer of a phosphoryl group from phosphoenolpyruvate (PEP) to ADP, generating ATP and plays a general role in caspase independent cell death of tumor cells.

PRDX1, also known as peroxiredoxin-1, has the Uniprot database accession number Q06830, June 1, 1994 - v1. It is involved in redox regulation of the cell.

OSTP, also known as osteopontin, has the Uniprot database accession number P10451, July 1, 1989 - v1. It acts as a cytokine involved in enhancing production of interferon-gamma and interleukin-12 and reducing production of interleukin-10 and is essential in the pathway that leads to type I immunity.

PDIA1, also known as protein disulfide-isomerase, has the Uniprot database accession number P07237, November 1, 1997 - v3. It catalyzes the formation, breakage and rearrangement of disulfide bonds.

MIF, also known as macrophage migration inhibitory factor, has the Uniprot database accession number P14174, January 23, 2007 - v4. It is involved in the innate immune response to bacterial pathogens.

CTNB1, also known as catenin beta-1, has the Uniprot database accession number P35222, February 1, 1994 - v1. It acts as a negative regulator of centrosome cohesion and blocks anoikis of malignant kidney and intestinal epithelial cells.

K2C8, also known as keratin, type II cytoskeletal 8, has the Uniprot database accession number P05787, January 23, 2007 - v7. Together with KRT19, helps to link the contractile apparatus to dystrophin at the costameres of striated muscle.

ANXA2, also known as annexin-2, has the Uniprot database accession number P07355, January 23, 2007 - v2. It is a calcium-regulated membrane-binding protein whose affinity for calcium is greatly enhanced by anionic phospholipids. It binds to calcium ions with high affinity and may be involved in heat-stress response.

CAPG, also known as macrophage-capping protein, has the Uniprot database accession number P40121, November 30, 2010 - v2. It is a calcium-sensitive protein which reversibly blocks the barbed ends of actin filaments but does not sever preformed actin filaments. It may play an important role in macrophage function.

FABP5, also known as Fatty acid-binding protein, epidermal, has the Uniprot database accession number Q01469, January 23, 2007 - v3. It shows high specificity for fatty acids and may be involved in keratinocyte differentiation.

MUC1, also known as mucin-1, has the Uniprot database accession number P15941, May 18, 2010 - v3. The alpha subunit has cell adhesive properties. It can act both as an adhesion and an anti-adhesion protein. May provide a protective layer on epithelial cells against bacterial and enzyme attack.

CAYP1, also known as calcyphosin, has the Uniprot database accession number Q13938, November 1, 1997 - v1. It is a calcium-binding protein that may play a role in cellular signaling events.

XPO2, also known as exportin-2, has the Uniprot database accession number P55060, March 29, 2005 - v3. Among others, this protein has been disclosed as exporting receptor for importin-alpha, mediating importin-alpha re-export from the nucleus to the cytoplasm after import substrates (cargos) and binding cooperatively to importin-alpha and to the GTPase Ran in its active GTP-bound form.

NGAL, also known as Neutrophil gelatinase-associated lipocalin, has the Uniprot database accession number P80188, November 1, 1995 - v2. It is involved in apoptosis due to interleukin-3 (IL3) deprivation and in innate immunity.

SG2A1, also known as mammaglobin-B, has the Uniprot database accession number 075556, November 1, 1998 - v1. It may bind androgens and other steroids.

ANXA1, also known as annexin-1, has the Uniprot database accession number P04083, January 23, 2007 - v2. It has been disclosed as playing an important role in the innate immune response, regulating the inflammatory process, having anti-inflammatory activity, and promoting resolution of inflammation and wound healing, among others.

HSPB1, also known as heat shock protein beta-1, has the Uniprot database accession number P04792, September 26, 2001 - v2. It is involved in stress resistance and actin organization.

PIGR, also known as polymeric immunoglobulin receptor, has the Uniprot database accession number P01833, June 26, 2007 - v4. This receptor binds polymeric IgA and IgM at the basolateral surface of epithelial cells.

CH10, also known as 10 kDa heat shock protein, mitochondrial, has the Uniprot database accession number P61604, January 23, 2007 - v2. It is essential for mitochondrial protein biogenesis, together with CPN60. Binds to CPN60 in the presence of Mg-ATP and suppresses the ATPase activity of the latter.

CD44, also known as CD44 antigen, has the Uniprot database accession number P16070, October 5, 2010 - v3. Mediates cell-cell and cell-matrix interactions through its affinity for HA, and possibly also through its affinity for other ligands such as osteopontin, collagens, and matrix metalloproteinases (MMPs).

CLIC1, also known as Chloride intracellular channel protein 1, has the Uniprot database accession number 000299, January 23, 2007 - v4. It inserts into membranes and form chloride ion channels. Channel activity depends on the pH. Membrane insertion seems to be redox-regulated and may occur only under oxydizing conditions. Involved in regulation of the cell cycle.

TPIS, also known as triosephosphate isomerase, has the Uniprot database accession number P60174, October 19, 2011 - v3. This protein is involved in the pathway gluconeogenesis, which is part of carbohydrate biosynthesis.

GSTP1, also known as glutathione S-transferase P, has the Uniprot database accession number P09211, January 23, 2007 - v2. It regulates negatively CDK5 activity via p25/p35 translocation to prevent neurodegeneration
GTR1 has the Uniprot database accession number P11166, October 3, 2006 - v2. It is a facilitative glucose transporter. This isoform may be responsible for constitutive or basal glucose uptake. It has a very broad substrate specificity; can transport a wide range of aldoses including both pentoses and hexoses
In another embodiment of the method of the first aspect of the invention, optionally in combination with any of the embodiments provided above or below, the method comprises (a) measuring, in vitro, in an isolated fluid sample from the female genital tract, the level of expression of the protein CLIC1, and the level of expression of one or more proteins selected from a second group consisting of: ENOA, KPYM, PDIA1, ANXA2 and FABP5; and (b) compared the level of expression of each of the tested proteins with a reference value; wherein if the proteins are overexpressed, it is indicative of endometrial carcinoma or bad prognosis.

In an attempt to improve the robustness of MMP9 as EC biomarker, the present inventors have surprisingly found that when MMP9 detection is combined with, CLIC1, a substantial improvement in sensitivity was achieved, reaching an AUC value of about up to 0.96. This finding was surprisingly because when MMP9 was combined with other proteins, the AUC resulting from the combination was unaffected or worse when compared with the one provided by MMP9 alone.

Thus, in one embodiment of any of the methods and uses provided by the present invention, above or below, it is determined the amount of MMP9 and CLIC1.

In any of the embodiments provided above or below, for any of the aspects of the invention, the level of expression is determined at the protein level. In this embodiment, the protein marker(s) include, but do not limit to, native-sequence polypeptides, isoforms, chimeric polypeptides, all homologs, fragments, and precursors of the markers, including modified forms of the polypeptides and derivatives thereof.

In any of the embodiments provided above or below, the level of expression is determined by immunochemistry.

The term "immunochemistry" as used herein refers to a variety of techniques for detecting antigens (usually proteins and peptides, and in the present case any of the proteins listed above alone or in combination) in a sample by exploiting the principle of antibodies binding specifically to said antigens. Visualising an antibody-antigen interaction can be accomplished in a number of ways. In the most common instance, an antibody is conjugated to an enzyme, such as peroxidase, that can catalyse a colour-producing reaction. Alternatively, the antibody can also be tagged to a fluorophore, such as fluorescein or rhodamine. The immunochemistry technique can be direct or indirect. The direct method is a one-step staining method and involves a labeled antibody (e.g. FITC-conjugated antiserum) reacting directly with the antigen. While this technique utilizes only one antibody and therefore is simple and rapid, the sensitivity is lower due to little signal amplification, such as with indirect methods, and is less commonly used than indirect methods. The indirect method involves an unlabeled primary antibody (first layer) that binds to the target antigen in the sample and a labeled secondary antibody (second layer) that reacts with the primary antibody. This method is more sensitive than direct detection strategies because of signal amplification due to the binding of several secondary antibodies to each primary antibody if the secondary antibody is conjugated to the fluorescent or enzyme reporter.

Further amplification can be achieved if the secondary antibody is conjugated to several biotin molecules, which can recruit complexes of avidin-, streptavidin or Neutravidin-enzyme. The indirect method, aside from its greater sensitivity, also has the advantage that only a relatively small number of standard conjugated (labeled) secondary antibodies needs to be generated. With the direct method, it would be necessary to label each primary antibody for every antigen of interest. It must be borne in mind that immunochemistry techniques can also be used to detect certain nucleic acid sequences if a tagged nucleic acid probe (designed to specifically bind to a certain target nucleic acid sequence) can later on be detected with a labelled antibody. Thus, the detection of the protein could be performed by using a tagged nucleic acid designed to bind a specific sequence of the target protein RNA, and then detecting said tagged nucleic acid with a labelled antibody which selectively binds to the tag.

Immunoassay procedures suitable include enzyme-linked immunosorbent assays (ELISA), enzyme immunodot assay, agglutination assay, antibody-antigen-antibody sandwich assay, antigen-antibody-antigen sandwich assay, immunocromatography, or other immunoassay formats well-known to the ordinarily skilled artisan.

In one embodiment, in combination with any of the embodiments provided above or below, the level of expression of protein is determined by an immunoassay.

In another embodiment, in combination with any of the embodiments provided above or below, the level of expression of protein is determined by ELISA.

Alternatively, the level of expression of protein can be determined by bioluminescence, fluorescence, chemiluminescence, electrochemistry, or mass spectrometry.

In another embodiment, in combination with any of the embodiments provided above or below, the level of expression of protein is determined using an antibody or a fragment thereof able to bind to the target protein(s).

The term "antibody or a fragment thereof able to bind to the target protein(s)" is to be understood as any immunoglobulin or fragment thereof able to selectively bind the target protein. It includes monoclonal and polyclonal antibodies. The term "fragment thereof encompasses any part of an antibody having the size and conformation suitable to bind an epitope of the target protein. Suitable fragments include F(ab), F(ab') and Fv. An "epitope" is the part of the antigen being recognized by the immune system (B-cells, T-cells or antibodies).

The antibodies used for specific detection can be polyclonal or monoclonal. There are well known means in the state of the art for preparing and characterizing antibodies. Methods for generating polyclonal antibodies are well known in the prior art. Briefly, one prepares polyclonal antibodies by immunizing an animal with the protein; then, serum from the immunized animal is collected and the antibodies isolated. A wide range of animal species can be used for the production of the antiserum. Typically the animal used for production of antisera can be a rabbit, mouse, rat, hamster, guinea pig or goat.

Moreover, monoclonal antibodies (MAbs) can be prepared using well-known techniques.

Typically, the procedure involves immunizing a suitable animal with the protein associated with the disease. The immunizing composition can be administered in an amount effective to stimulate antibody producing cells. Methods for preparing monoclonal antibodies are initiated generally following the same lines as the polyclonal antibody preparation. The immunogen is injected into animals as antigen. The antigen may be mixed with adjuvants such as complete or incomplete Freund's adjuvant. At intervals of two weeks, approximately, the immunization is repeated with the same antigen.

In another particular embodiment of the third aspect, the means to carry out the invention form part of a kit. The antibody or fragment thereof for detecting the target protein(s) can be included in a kit. The kit may additionally comprise means (additives, solvents) to visualize the antibody-protein interactions.

These antibodies can be used as "means" for determining the expression of the target proteins in the fifth aspect of the invention.

All the embodiments provided above, under the first aspect of the invention, regarding the proteins to be analysed (from 2 to 10 of the list and the protein sets comprising 2, 3, 4, 5, or 6 particular markers), are also particular embodiments of the use of the third aspect of the invention.

Alternatively, the level of expression is determined at the mRNA level.

In one embodiment, the amount of mRNA of each one of the markers are detected via polymerase chain reaction using, for example, oligonucleotide primers that hybridize to one or more polynucleotide endometrial cancer markers or complements of such polynucleotides. Within other embodiments, the amount of mRNA is detected using a hybridization technique, employing oligonucleotide probes that hybridize to one or more polynucleotide endometrial cancer markers or complements of such polynucleotides.

When using mRNA detection, the method may be carried out by combining isolated mRNA with reagents to convert to cDNA according to standard methods well known in the art, treating the converted cDNA with amplification reaction reagents (such as cDNA PCR reaction reagents) in a container along with an appropriate mixture of nucleic acid primers; reacting the contents of the container to produce amplification products; and analyzing the amplification products to detect the presence of one or more of the polynucleotide endometrial cancer markers in the sample. For mRNA, the analyzing step may be accomplished using Northern Blot analysis to detect the presence of polynucleotide endometrial cancer markers in the sample. The analysis step may be further accomplished by quantitatively detecting the presence of polynucleotide endometrial cancer markers in the amplification product, and comparing the quantity of marker detected against a panel of expected values for the known presence or absence of such markers in normal and malignant tissue derived using similar primers.

In another embodiment, the invention provides a method wherein mRNA is detected by: (a) isolating mRNA from a sample and combining the mRNA with reagents to convert it to cDNA; (b) treating the converted cDNA with amplification reaction reagents and nucleic acid primers that hybridize to one or more of the polynucleotide endometrial cancer markers endometrial cancer marker to produce amplification products; (c) analyzing the amplification products for determining the amount of mRNA present encoding the protein endometrial cancer marker; and (d) comparing the determined amount of mRNA to an amount detected against a panel of expected values for normal and diseased tissue (e.g. , malignant tissue) derived using similar methods.

In particular embodiments of the invention, RT-PCR can be used to amplify the mRNA for protein endometrial cancer markers for detection and analysis. Other embodiments of the invention use quantitative RT-PCR to quantitatively determine amount of mRNA for protein endometrial cancer markers. Further embodiments of the invention use real time RT-PCR for quantification and analysis.

In a fourth aspect, the present invention provides a kit.

In one embodiment of the fourth aspect of the invention, the means for determining the level of expression are antibody(ies) or fragments thereof that specifically bind(s) to the target protein(s).

The number of specific antibodies or fragments thereof included in the kit will depend on the number of proteins to be detected. In this regard, previous embodiments of the method of the first aspect of the invention have provided several sets of proteins to be determined for performing an appropriate diagnosis or prognosis of endometrial carcinoma, these set of proteins comprising two, three, four, five, six seven, eight, nine or ten proteins. Starting from this information, the skilled person can be able of choosing one of the sets previously mentioned and select the more appropriate antibody or fragment thereof, from those already available, for the detection of each protein. The incorporation of the selected antibodies in the appropriate solid support can be performed using routine methods.

In another embodiment, the present invention provides a kit comprising a solid support and means for detecting the level of expression MMP9 and CLIC1.

In another embodiment of the fourth aspect of the invention, the kit is an ELISA kit. In this embodiment, the kit comprises a solid support and means for determining the level of expression of any of the sets of proteins provided above. In another embodiment, the kit comprises a solid support and antibodies or fragments thereof which specifically bind to the target proteins to be detected, these antibodies being conjugated with a reporter molecule capable of producing a signal.

The "solid support" includes a nitrocellulose membrane, glass or a polymer. The most commonly used polymers being cellulose, polyacrylamide, nylon, polystyrene, polyvinyl chloride or polypropylene. The solid supports may be in the form of strips, tubes, beads, discs or microplates, or any other surface suitable for conducting an immunoassay.

The "reporter molecule" as used in the present specification is meant a molecule which, by its chemical nature, provides an analytically identifiable signal which allows the detection of antigen-bound antibody. Detection may be either qualitative or quantitative. The most commonly used reporter molecules in this type of assay are either enzymes, fluorophores or radionuclide containing molecules (i.e., radioisotopes). In the case of an enzyme immunoassay, an enzyme is conjugated to the second antibody, generally by means of glutaraldehyde or periodate. As will be readily recognized, however, a wide variety of different conjugation techniques exist, which are readily available to those skilled in the art. Commonly used enzymes include horseradish peroxidase, glucose oxidase, β-galactosidase and alkaline phosphatase, among others. The substrates to be used with the specific enzymes are generally chosen for the production, upon hydrolysis by the corresponding enzyme, of a detectable colour change. For example, 5-bromo-4-chloro-3-indolyl phosphate/nitroblue tetrazolium is suitable for use with alkaline phosphatase conjugates; for peroxidase conjugates, 1,2-phenylenediamine, 5-aminosalicylic acid, 3,3:5,5:tetra methyl benzidine or tolidine are commonly used. It is also possible to employ fluorogenic substrates, which yield a fluorescent product rather than the chromogenic substrates noted above. Examples of fluorogenic substrates are fluorescein and rhodamine. When activated by illumination with light of a particular wavelength, the fluorochrome-labelled antibody absorbs the light energy, inducing a state of excitability in the molecule, followed by emission of the light at a characteristic colour visually detectable with a light microscope. Immunofluorescence and EIA techniques are both well established in the art and are particularly preferred for the present method. However, other reporter molecules, such as radioisotope, chemiluminescent, and bioluminescent molecules and/or dyes and other chromogenic substances, may also be employed.

The choice of a particular reporter molecule conjugated antibody will be, for the most part, determined by the intended use and user of the test kit of the present invention.

In another embodiment, the kit is a microarray.

In another embodiment, the kit is a microarray including a defined set of genes encoding protein endometrial cancer markers. All the embodiments provided above for particular sets of proteins with 2, 3, 4, 5, 6, 7, 8, 9, or 10 proteins, whose expression is significantly altered by endometrial disease, are also particular embodiments of microarrays.

The in vitro methods of the invention provide diagnostic and prognostic information. In one embodiment, the methods of the invention further comprise the steps of (i) collecting the diagnostic or prognostic information, and (ii) saving the information in a data carrier.

In the sense of the invention a "data carrier" is to be understood as any means that contain meaningful information data for the diagnosis or prognosis of endometrial carcinoma, such as paper. The carrier may also be any entity or device capable of carrying the prognosis data. For example, the carrier may comprise a storage medium, such as a ROM, for example a CD ROM or a semiconductor ROM, or a magnetic recording medium, for example a floppy disc or hard disk. Further, the carrier may be a transmissible carrier such as an electrical or optical signal, which may be conveyed via electrical or optical cable or by radio or other means. When the prognosis data are embodied in a signal that may be conveyed directly by a cable or other device or means, the carrier may be constituted by such cable or other device or means. Other carriers relate to USB devices and computer archives. Examples of suitable data carrier are paper, CDs, USB, computer archives in PCs, or sound registration with the same information.

Throughout the description and claims the word "comprise" and variations of the word are not intended to exclude other technical features, additives, components, or steps. Furthermore, the word "comprise" and its variations encompasses the term "consisting of". Additional objects, advantages and features of the invention will become apparent to those skilled in the art upon examination of the description or may be learned by practice of the invention. The following examples are provided by way of illustration, and they are not intended to be limiting of the present invention. Furthermore, the present invention covers all possible combinations of particular and preferred embodiments described herein.

### EXAMPLES

### Example 1

### Reagents

Albumin and IgG Depletion SpinTrap columns were purchased from GE Healthcare (cat.no. 28-9480-20). Lys C endoproteinase MS grade was purchased from Thermo Scientific (cat.no. 90051). Solid phase extraction cartridges, Sep Pak tC18, 50 mg, were obtained from Waters (cat.no.WAT054960). All other reagents were obtained from Sigma-Aldrich.

### Patients and sample collection

A total of 38 patients (20 women suffering from EC and 18 non-EC controls, i.e., women having EC symptoms but not diagnosed with EC) participating in this prospective study were recruited in the Vall d'Hebron University Hospital (Barcelona, Spain) during 2012 to 2015. Informed consent forms, approved by the Vall d'Hebron Ethical Committee, were signed by all patients (approval number: PR_AMI_50-2012).

Uterine fluid samples were collected by aspiration with a Cornier Pipelle (Eurogine Ref. 03040200) in the office of the clinician or in the operating room prior to surgery and transferred to 1.5 ml microtubes. Phosphate buffer saline was added in a 1:1 (v/v) ratio and centrifuged at 2,500 ref for 20 min in order to separate the soluble fraction (supernatant) from the solid fraction (pellet). The separated fractions were kept at -80°C until use.

### Sample preparation for the verification study

Supernatants from uterine aspirates coming from 20 EC patients and 18 non-EC controls were sonicated to disrupt potential microvesicles, protein aggregates, and/or mucus by 5 cycles at 100% amplitude during 5 seconds (Labsonic M). Albumin and immunoglobulin G were then depleted from 50 µl of supernatant samples using the Albumin & IgG depletion spin trap kit according to the manufacturer's instructions. Total protein concentration was measured by the Bradford assay performed in triplicate. Each of the 38 samples was then separated into two aliquots of 25 µg to generate duplicates for the whole process, with exception of one sample for which the amount of material was not sufficient for duplication. The samples were diluted into a 50 mM solution of ammonium bicarbonate to a final volume of 120 µl and were denatured by addition of 185 µl of 10 M urea suspended in 50 mM ammonium bicarbonate, incubated at 22°C under agitation for 20 min, and followed by 10 min incubation in an ultrasonic bath (Branson 5510). The samples were then reduced with 7.8 µl of 200 mM dithiothreitol for 60 min at 37°C, and alkylated with 12.2 µl of 400 mM iodoacetamide at 22°C for 30 min in the dark. The samples were digested for 4 h at 37°C with Lys C (protease/total protein amount ratio of 1/150; w/w). Afterwards, the concentration of urea was diluted to 1 M with 50 mM ammonium bicarbonate buffer, and samples were incubated overnight at 37°C with trypsin (protease/total protein amount ratio of 1/50; w/w). The trypsin activity was quenched by addition of 1 µl of neat formic acid per 100 µl of solution. The samples were spiked with the mix of heavy synthetic peptides and then desalted onto solid phase extraction cartridges (Sep Pak tC18, 50mg, Waters). The eluates were subsequently evaporated to dryness in a vacuum centrifuge and then resuspended in 0.1% formic acid before LC-PRM analysis.

### LC-MSIMS PRM configuration

The LC MS setup consisted of a Dionex Ultimate 3000 RSLC chromatography system configured for a high-pressure binary gradient and operated in column switching mode. The mobile phase A consisted of 0.1% formic acid in water, the phase B in 0.1% formic acid in acetonitrile and the loading phase in 0.05% trifluoroacetic acid and 1% acetonitrile in water. The equivalent of 250 ng of each digested sample was injected and loaded onto a trap column (75 µm × 2 cm, C18 pepmap 100, 3µm) at 5 µl/min and further eluted onto the analytical column (75 µm × 15 cm, C18 pepmap 100, 2µm) at 300 nl/min by a linear gradient starting from 2 % A to 35 % B in 48 min. The MS analysis was performed by a hybrid quadrupole orbitrap mass spectrometer (Q Exactive plus, Thermo Scientific) operated in PRM mode. The MS cycle started with a full MS1 scan performed at a resolving power of 70,000 (at 200 m/z) followed by time scheduled targeted PRM scans acquired at a resolving power of 35,000 (at 200 m/z) with a normalized collision energy of 20. The quadrupole isolation window for the PRM events were set to 1 m/z unit and the duration of the time scheduled windows for each pair of endogenous and isotopically labeled peptides were set to 2 min.

### Statistical analysis

All analyses were performed in SPSS version 20.0 (IBM, USA) and Graph Pad Prism v.6.0 (GraphPad Software, CA, USA). The averaged light/heavy area ratios were calculated between duplicates. The linear correlation between the signature peptides of the same protein was calculated using the Pearson correlation coefficient. Due to the non-normally distributed dataset, evaluated by Kolmogorov-Smirnova and Shapiro-Wilk tests, comparison of the expression of the monitored peptides between tumor and control samples was assessed by the nonparametric Mann-Whitney U test. P-values lower than 0.05 along with fold changes over 3 were considered statistically significant. Receiver operating characteristic (ROC) curves were used to calculate the relationship between sensitivity and specificity for EC versus the non-EC control group and hence to evaluate the diagnostic performance for each biomarker candidate.

### Results

Expression of each biomarker candidate between 20 EC patients and 18 non-EC controls was compared. Importantly, both patients and controls were postmenopausal women suffering from an abnormal vaginal bleeding, as 93% of patients suffering from EC present these clinical features but only 15% of those will be finally diagnosed with EC.

Based on the Bradford assays, 250 ng of the total protein concentration after albumin and IgGs depletion was injected for each sample. The constant amount of injected protein among samples was further confirmed by the integration of the total ion chromatogram of the MS1 scans. After MS data curation, the relative levels (light/heavy ratios) of the 98 monitored peptides in MS2 were subjected to Mann Whitney test for their comparison between tumor and control samples. 58 peptides corresponding to 32 proteins showed significant differences between the two groups with p-value <0.05 and fold change larger than 1.5: PERM, CADH1, SPIT1, ENOA, MMP9, LDHA, CASP3, KPYM, PRDX1, OSTP, PDIA1, NAMPT, MIF, CTNB1, K2C8, ANXA2, CAPG, FABP5, MUC1, CAYP1, XPO2, NGAL, SG2A1, ANXA1, HSPB1, PIGR, CH10, CD44, CLIC1, TPIS, GSTP1, and GTR1 . All these proteins were overexpressed in tumor samples as compared to control samples.

To further evaluate their utility as biomarkers for EC diagnosis, a ROC analysis to determine the sensitivity and specificity of each biomarker was performed. Interestingly, all proteins achieved an excellent Area Under the Curve (AUC) values for defining an increased likelihood of EC in minimally invasive uterine aspirates, ranging from 0.71 to 0.95. The 10 best-performing individual proteins were PERM, CADH1, SPIT1, ENOA, MMP9, LDHA, CASP3, KPYM isoform M1-M2, PRDX1 and OSTP isoform A, all of them with AUC values higher than 0.9.

| **Uniprot Accession Number** | **Protein ID** | **Fold Change** | **Adjusted P-value** | **AUC** |
|---|---|---|---|---|
| P05164 | PERM | 13,3 | 1,E-04 | 0,95 |
| P12830 | CADH1 | 3,8 | 9,E-05 | 0,94 |
| O43278 | SPIT 1 | 3,3 | 1,E-04 | 0,93 |
| P06733 | ENOA | 3,8 | 1,E-04 | 0,92 |
| P14780 | MMP9 | 5,7 | 1,E-04 | 0,91 |
| P00338 | LDHA | 5,7 | 1,E-04 | 0,91 |
| P42574 | CASP3 | 4,9 | 2,E-04 | 0,91 |
| P14618 | KPYM_Isoform M1-M2 | 5,4 | 1,E-04 | 0,91 |
| Q06830 | PRDX1 | 4,2 | 2,E-04 | 0,90 |
| P10451 | OSTP_Isoform A | 11,4 | 2,E-04 | 0,90 |
| P07237 | PDIA1 | 3,0 | 3,E-04 | 0,88 |
| P43490 | NAMPT | 4,0 | 3,E-04 | 0,88 |
| P14174 | MIF | 3,1 | 3,E-04 | 0,87 |
| P35222 | CTNB1 | 4,2 | 3,E-04 | 0,87 |
| P05787 | K2C8 | 3,6 | 3,E-04 | 0,88 |
| P07355 | ANXA2 | 4,8 | 4,E-04 | 0,87 |
| P40121 | CAPG | 3,5 | 6,E-04 | 0,85 |
| Q01469 | FABP5 | 3,9 | 6,E-04 | 0,85 |
| P15941 | MUC1 | 3,6 | 1,E-03 | 0,84 |
| Q13938 | CAYP1 | 3,4 | 1,E-03 | 0,83 |
| P55060 | XPO2 | 4,0 | 1,E-03 | 0,83 |
| P80188 | NGAL | 4,4 | 4,E-03 | 0,79 |
| O75556 | SG2A1 | 3,2 | 5,E-03 | 0,78 |
| P04083 | ANXA1 | 3,9 | 7,E-03 | 0,77 |
| P04792 | HSPB1 | 3,1 | 4,E-03 | 0,79 |
| P01833 | PIGR | 3,4 | 7,E-03 | 0,77 |
| P61604 | CH10 | 2,3 | 5,E-03 | 0,77 |
| P16070 | CD44 | 2,6 | 1,E-04 | 0,86 |
| O00299 | CLIC1 | 2,8 | 2,E-04 | 0,86 |
| P60174 | TPIS | 2,9 | 1,E-04 | 0,87 |
| P09211 | GSTP1 | 2,7 | 1,E-03 | 0,81 |
| P11166 | GTR1 | 1,5 | 3,E-02 | 0,71 |

These results allow concluding that these proteins present very high sensitivity and specificity in isolated fluid samples from the female genital tract.

Furthermore, a bioinformatic analysis using Ingenuity Pathway Analysis (IPA) to better understand the association of these proteins with cancer and their origin regarding the subcellular location was also performed. As expected, integration of the data resulted in the identification of cancer, inflammatory disease, organismal injury and abnormalities, and reproductive system disease as the top diseases associated to these biomarkers. The top five molecular and cellular functions involved with these proteins included cellular movement, cellular death and survival, cellular development, cellular growth and proliferation, and cell to cell signaling and interaction, all of them important processes altered in cancer. These proteins are mainly found in the cytoplasm, plasma membrane and extracellular space, indicating that they are coming either from secretion of the epithelial and inflammatory cells of the endometrium or by necrosis of cells in the proximal tissue. These features were crucial to facilitate the use of those biomarkers to diagnose EC in proximal body fluids related to the female genital tract.

### Example 2

The diagnostic performance of several proteins was evaluated by liquid chromatography with mass spectrometry detection using parallel reaction monitoring acquisition method (LC-PRM), as disclosed above, in uterine aspirate samples from 116 women.

The processing of uterine aspirates included its collection by aspiration with a specialized device, and dilution of the aspirate in a tube with a PBS1x saline solution in a 1:1 (v/v) ratio. Then, centrifugation at 2,500 x g for 20 min was performed in order to separate the liquid fraction from the cellular fraction. The supernatants of uterine aspirates were used to assess the protein biomarkers.

From the 116 women, 69 were diagnosed with EC, including 49 endometrioid EC (EEC) and 20 non-endometrioid serous ECs (SEC); and the remaining 47 women were non-EC women with normal endometrium or diagnosed with benign disorders.

To obtain significant EC biomarkers, the expression of each marker, which was measured as the light/heavy area ratio obtained in the LC-PRM study (LC-PRM configuration is the same as in example 1), was compared in the tumor population (n=69) against the non-EC population (n=47) by using the nonparametric Mann-Whitney U test. Adjusted p-values lower than 0.05 were considered statistically significant. Receiver operating characteristic (ROC) analysis was used to assess the specificity and sensitivity of the biomarkers and the area under the ROC curve (AUC) was estimated for each individual protein. The results are summarized in Table 2 below:

| **COHORT 1 (n=116)** | | | |
|---|---|---|---|
| **Protein ID** | **FC All T/ C** | **A\|Adjusted value** | **P AUC** |
| **LDHA** | 5.49 | 1.E-11 | 0.91 |
| **KPYM:Isoform M1-M2** | 5.67 | 1.E-11 | 0.90 |
| ****KPYM:Isoform M1-M3** | 3.39 | 9.E-05 | 0.72 |
| **MMP9** | 11.40 | 2.E-11 | 0.89 |
| **NAMPT** | 3.84 | 4.E-11 | 0.88 |
| **SPIT1** | 3.92 | 5.E-11 | 0.88 |
| **CADH1** | 3.33 | 5.E-11 | 0.88 |
| **ENOA** | 3.66 | 1.E-10 | 0.87 |
| **PERM** | 8.39 | 4.E-10 | 0.86 |
| **CAPG** | 3.74 | 8.E-10 | 0.85 |
| **CH10** | 3.08 | 1.E-09 | 0.85 |
| **CTNB1** | 3.89 | 2.E-09 | 0.84 |
| **K2C8** | 3.02 | 2.E-09 | 0.84 |
| **CLIC1** | 2.91 | 4.E-09 | 0.84 |
| **PDIA1** | 2.68 | 4.E-09 | 0.83 |
| **PRDX1** | 2.85 | 5.E-09 | 0.83 |
| **CD44** | 2.71 | 6.E-09 | 0.83 |
| **MIF** | 2.85 | 9.E-09 | 0.83 |
| **FABP5** | 3.19 | 1.E-08 | 0.82 |
| **XPO2** | 4.68 | 2.E-08 | 0.81 |
| **TPIS** | 2.35 | 7.E-08 | 0.80 |
| **CASP3** | 3.49 | 1.E-07 | 0.80 |
| **GSTP1** | 2.88 | 5.E-07 | 0.79 |
| **ANXA1** | 4.06 | 7.E-07 | 0.78 |
| **NGAL** | 3.63 | 3.E-06 | 0.77 |
| **ANXA2** | 3.22 | 3.E-06 | 0.76 |
| **GTR1** | 3.17 | 6.E-06 | 0.76 |
| **OSTP: Isoform A, B, D** | 2.28 | 7.E-06 | 0.76 |
| **MUC1** | 2.38 | 7.E-06 | 0.76 |
| ** Different isoform of the same protein showing different performance | | | |
| T: EC cases | | | |
| C: non-EC controls | | | |
| FC:fold change | | | |

From the more robust biomarkers, the inventors paid attention in improving the diagnostic information provided by MMP9. To this end, a logistic regression model was adjusted to the data in order to assess the power of the different combinations of proteins to classify samples in two clinical categories (cancer and control). ROC curves were generated for each of these regression models; the AUC, the sensitivity and specificity at the "optimal" cutoff point for discrimination between groups were obtained. The optimal cut-off corresponded to the threshold that maximized the distance to the identity (diagonal) line. The optimality criterion was: max (sensitivities + specificities). AUCs 95% confidence intervals (CI) were computed with the Delong's method (20). The 95% CIs of the sensitivity and specificity values were computed with bootstrap resampling and the averaging methods described by Fawcett (Fawcett T., "An Introduction to ROC Analysis", Pattern Recogn Lett. 2006, v. 27, pages 861-874). All ROC analysis were performed using the R "pROC" package (Robin X. et al., "pROC: an open-source package for R and S+ to analyze and compare ROC curves", BMC Bioinformatics, 2011, v. 12, page 77). To assess the robustness of each protein panel, the "leave-one-out" cross-validation procedure was performed by applying to each sample in the dataset the logistic regression model adjusted to the remaining samples on the dataset, hence deriving a new ROC curve and afterwards performing the usual ROC analysis. In a similar way, the discrimination power of the diagnostic protein panel was further validated by applying to each sample of an independent set of samples (cohort 2: cohort in example 1) the logistic regression model adjusted to the initial set (cohort 1: cohort in example 2), hence deriving a new ROC curve and afterwards performing the usual ROC analysis.

Thus, it was found that MMP9 biomarker value was remarkably improved when its determination was performed in combination with KPYM, ENOA, PRDX1, MIF, GSTP1, CAPG, CADH1, HSPB1, PDIA1, LDHA, CLIC1, CASP3, FABP5, TPIS, LDHA, CTNB1, CH10, NAMPT, and ANXA2; contrary to other proteins which, when combined with MMP9, adversely affected the EC biomarker value of MMP9 alone.

A specific example of a positive combination is the combination MMP9+KPYM which showed an AUC value of 0.96. This finding was surprising because MMP9 and KPYM have an individual AUC value of 0.89 and 0.90, respectively, and, when combined, MMP9' AUC value is increased.

On the contrary, the combination of MMP9 with PERM did not improve the accuracy to detect EC. In example 1 and 2, the individual AUC values obtained for MMP9 were 0.91 and 0.89; and the AUC values for PERM were 0.96 and 0.86. However, their combination did not report any improved AUC value. Combination of MMP9 with PERM has an AUC value of 0.89.

### Example 3

Detection of MMP9 and KPYM through ELISA technology. ELISA kits (R&D Systems and USCN life Science and Technology Company, respectively) according to the manufacturer's protocol. For MMP9, 105 uterine aspirate samples were analyzed using 1:10; 1:100 or 1:1000 dilutions. For KPYM, only 39 uterine aspirate samples could be analyzed using 1:2, 1:4 or 1:10 dilutions due to a lack of sample material. All samples were assayed in duplicates and the average values were reported as ng/mL. The linear correlation between the results from LC-PRM and ELISA assays was calculated using the Pearson correlation coefficient. It was found that ELISA results were highly correlated with those observed in mass spectrometry. Thus, MMP9 and KPYM could be used in antibody-based techniques to diagnose EC.

## Claims

1. A method of diagnosis or prognosis endometrial carcinoma, the method comprising determining the level of expression of the protein CLIC1 in uterine aspirate fluid isolated from the female genital tract.

2. A method for identifying a subject suspicious of suffering from endometrial carcinoma, the method comprising:
a) determining, in vitro, the level of expression of the protein CLIC1, in uterine aspirate fluid isolated from the female genital tract, and
b) comparing the level of step (a) with a reference control level,
wherein if the level determined in step (a) is higher than the reference control level, it is indicative that the subject is suspicious of suffering endometrial carcinoma.

3. A method of deciding or recommending whether to initiate a medical regimen of a subject suspicious of suffering endometrial carcinoma, which method comprises the steps of:
a) determining, in vitro, the level of expression of the protein CLIC1, in uterine aspirate fluid isolated from the female genital tract; and
b) diagnosing the endometrial carcinoma or determining whether the subject is suspicious of suffering endometrial carcinoma, if the protein level in the test sample is higher than a reference control level;
wherein:
i) if the subject is diagnosed of suffering from endometrial carcinoma, or of being suspicious of suffering from endometrial carcinoma, then the initiation of the medical regimen is recommended, and
ii) if the patient is diagnosed of not suffering from endometrial carcinoma, the follow-up is performed optionally in consideration of the result of an examination of the patient by a physician.

4. A method for determining the efficacy of a medical regimen in a patient already diagnosed of endometrial carcinoma, the method comprising the steps of:
(a) in vitro measuring the level of expression of the protein CLIC1, in uterine aspirate fluid isolated from the female genital tract prior to the administration of the medical regimen;
(b) in vitro measuring the level of said marker in uterine aspirate fluid from the subject's female genital tract once started the administration of the medical regimen; and
(c) comparing the levels measured in steps (a) and (b), in such a way that if the level measured in step (b) is lower than the level measured in step (a), it is indicative that the medical regimen is effective in the treatment of endometrial carcinoma; or, alternatively, the method comprising the steps of:
(i) in vitro measuring the level of expression of the protein CLIC1 in uterine aspirate fluid isolated from the subject's female genital tract once started the administration of the medical regimen; and
(ii) comparing the level measured in step (i) with a reference control level of the marker, wherein, if the level measured in step (i) is not higher than the reference control level, it is indicative that the medical regimen is effective in the treatment of endometrial carcinoma.

5. The method according to any of the preceding claims, wherein the level of expression is determined at the protein level.

6. The method according to claim 5, wherein the level of expression of protein is determined using an antibody or a fragment thereof able to bind to the protein.

7. The method according to claim 6, wherein said antibody or fragment o thereof forms part of a kit.

8. The method according to any of the preceding claims, further comprising (i) collecting the diagnostic or prognostic information, and (ii) saving the information in a data carrier.

9. Use of the marker CLIC1 as an in vitro marker for diagnosing or prognosing endometrial carcinoma in uterine aspirate fluid isolated from the female genital tract.

10. Use of means for determining the level of the marker CLIC1 in the method as defined in any of the preceding claims.

## Patentansprüche

1. Verfahren zur Diagnose oder Prognose von Endometriumkarzinom, wobei das Verfahren das Bestimmen des Expressionsniveaus des Proteins CLIC1 in Gebärmutteraspiratfluid isoliert aus dem weiblichen Genitaltrakt umfasst.

2. Verfahren zur Identifizierung eines Individuums, von welchem es vermutet wird unter Endometriumkarzinom zu leiden, wobei das Verfahren Folgendes umfasst:
a) das, *In-vitro-,* Bestimmen des Expressionsniveaus des Proteins CLIC1 in Gebärmutteraspiratfluid isoliert aus dem weiblichen Genitaltrakt, und
b) das Vergleichen des Niveaus aus Schritt (a) mit einem Referenzkontrollniveau,
wobei, wenn das in Schritt (a) bestimmte Niveau höher als das Referenzkontrollniveau ist, dies darauf schließen lässt, dass es vermutet wird, dass das Individuum unter Endometriumkarzinom leidet.

3. Verfahren zum Entscheiden oder Empfehlen, ob man mit einem medizinischen Regime eines Individuums, von welchem es vermutet wird unter Endometriumkarzinom zu leiden, beginnen soll, welches Verfahren die folgenden Schritte umfasst:
a) das, *In-vitro-,* Bestimmen des Expressionsniveaus des Proteins CLIC1 in Gebärmutteraspiratfluid isoliert aus dem weiblichen Genitaltrakt; und
b) das Diagnostizieren des Endometriumkarzinoms oder das Bestimmen, ob es vermutet wird, dass das Individuum unter Endometriumkarzinom leidet, wenn das Proteinniveau in der Testprobe höher als ein Referenzkontrollniveau ist;
wobei:
i) wenn das Individuum diagnostiziert wird, dass es unter Endometriumkarzinom leidet, oder wenn man vermutet, dass es unter Endometriumkarzinom leidet, dann der Beginn des medizinischen Regimes empfohlen wird, und
ii) wenn die Patientin diagnostiziert wird, dass sie nicht unter Endometriumkarzinom leidet, die Weiterverfolgung wahlweise unter Berücksichtigung des Ergebnisses einer Untersuchung der Patientin vonseiten eines Arztes durchgeführt wird.

4. Verfahren zur Bestimmung der Wirksamkeit eines medizinischen Regimes in einer Patientin, welche bereits mit Endometriumkarzinom diagnostiziert worden ist, wobei das Verfahren die folgenden Schritte umfasst:
(a) das *In-vitro-Messen* des Expressionsniveaus des Proteins CLIC1 in Gebärmutteraspiratfluid isoliert aus dem weiblichen Genitaltrakt vor der Verabreichung des medizinischen Regimes;
(b) das *In-vitro-Messen* des Niveaus des genannten Markers in Gebärmutteraspiratfluid aus dem weiblichen Genitaltrakt des Individuums, wenn einmal mit der Verabreichung des medizinischen Regimes begonnen worden ist; und
(c) das Vergleichen der in den Schritten (a) und (b) gemessenen Niveaus, derart dass, wenn das in Schritt (b) gemessene Niveau niedriger als das in Schritt (a) gemessene Niveau ist, dies darauf schließen lässt, dass das medizinische Regime bei der Behandlung von Endometriumkarzinom wirksam ist; oder, alternativ, wobei das Verfahren die folgenden Schritte umfasst:
(i) das *In-vitro-Messen* des Expressionsniveaus des Proteins CLIC1 in Gebärmutteraspiratfluid isoliert aus dem weiblichen Genitaltrakt des Individuums, wenn einmal mit der Verabreichung des medizinischen Regimes begonnen worden ist; und
(ii) das Vergleichen des in Schritt (i) gemessenen Niveaus mit einem Referenzkontrollniveau des Markers, wobei, wenn das in Schritt (i) gemessene Niveau nicht höher als das Referenzkontrollniveau ist, dies darauf schließen lässt, dass das medizinische Regime bei der Behandlung von Endometriumkarzinom wirksam ist.

5. Verfahren nach einem der vorhergehenden Ansprüche, wobei das Expressionsniveau auf Proteinebene bestimmt wird.

6. Verfahren nach Anspruch 5, wobei das Expressionsniveau des Proteins unter Verwendung eines Antikörpers oder eines Fragment desselben, welcher/welches in der Lage ist, sich an das Protein zu binden, bestimmt wird.

7. Verfahren nach Anspruch 6, wobei der genannte Antikörper oder das genannte Fragment desselben Bestandteil eines Kits ist.

8. Verfahren nach einem der vorhergehenden Ansprüche, zusätzlich umfassend (i) das Sammeln der diagnostischen oder prognostischen Information, und (ii) das Speichern der Information in einem Datenträger.

9. Verwendung des Markers CLIC1 als *In-vitro-Marker* zur Diagnose oder Prognose von Endometriumkarzinom in Gebärmutteraspiratfluid isoliert aus dem weiblichen Genitaltrakt.

10. Verwendung von Mitteln zur Bestimmung des Niveaus des Markers CLIC1 im Verfahren nach einem der vorhergehenden Ansprüche.

## Revendications

1. Procédé de diagnostic ou de pronostic d'un carcinome de l'endomètre, le procédé comprenant la détermination du niveau d'expression de la protéine CLIC1 dans du liquide d'aspiration utérine isolé à partir de l'appareil génital féminin.

2. Procédé pour identifier un sujet suspecté d'être atteint de carcinome de l'endomètre, le procédé comprenant :
a) déterminer, *in vitro,* le niveau d'expression de la protéine CLIC1, dans du liquide d'aspiration utérine isolé à partir de l'appareil génital féminin, et
b) comparer le niveau de l'étape (a) avec un niveau de contrôle de référence,
dans lequel, si le niveau déterminé dans l'étape (a) est supérieur au niveau de contrôle de référence, ceci est indicatif du fait que le sujet est suspecté d'être atteint de carcinome de l'endomètre.

3. Procédé pour décider ou recommander si initier un régime médical d'un sujet suspecté d'être atteint de carcinome de l'endomètre, procédé qui comprend les étapes consistant à :
a) déterminer, *in vitro,* le niveau d'expression de la protéine CLIC1, dans du liquide d'aspiration utérine isolé à partir de l'appareil génital féminin ; et
b) diagnostiquer le carcinome de l'endomètre ou déterminer si le sujet est suspecté d'être atteint de carcinome de l'endomètre, si le niveau de protéine dans l'échantillon de test est supérieur à un niveau de contrôle de référence ;
dans lequel :
i) si le sujet est diagnostiqué comme étant atteint de carcinome de l'endomètre, ou étant suspecté d'être atteint de carcinome de l'endomètre, l'initiation du régime médical est alors recommandée, et
ii) si la patiente est diagnostiquée comme n'étant pas atteinte de carcinome de l'endomètre, le suivi est optionnellement effectué en prenant compte du résultat d'un examen de la patiente par un médecin.

4. Procédé pour déterminer l'efficacité d'un régime médical chez une patiente déjà diagnostiquée de carcinome de l'endomètre, le procédé comprenant les étapes consistant à :
(a) mesurer *in vitro* le niveau d'expression de la protéine CLIC1, dans du liquide d'aspiration utérine isolé à partir de l'appareil génital féminin avant l'administration du régime médical ;
(b) mesurer *in vitro* le niveau dudit marqueur dans du liquide d'aspiration utérine à partir de l'appareil génital féminin du sujet une fois commencée l'administration du régime médical ; et
(c) comparer les niveaux mesurés dans les étapes (a) et (b), de telle sorte que, si le niveau mesuré dans l'étape (b) est inférieur au niveau mesuré dans l'étape (a), ceci est indicatif que le régime médical est efficace dans le traitement de carcinome de l'endomètre ; ou, alternativement, le procédé comprenant les étapes consistant à :
(i) mesurer *in vitro* le niveau d'expression de la protéine CLIC1 dans du liquide d'aspiration utérine isolé à partir de l'appareil génital féminin du sujet une fois commencée l'administration du régime médical ; et
(ii) comparer le niveau mesuré dans l'étape (i) avec un niveau de contrôle de référence du marqueur, dans lequel, si le niveau mesuré dans l'étape (i) n'est pas supérieur au niveau de contrôle de référence, ceci est indicatif que le régime médical est efficace dans le traitement de carcinome de l'endomètre.

5. Procédé selon l'une quelconque des revendications précédentes, dans lequel le niveau d'expression est déterminé au niveau de protéine.

6. Procédé selon la revendication 5, dans lequel le niveau d'expression de protéine est déterminé en utilisant un anticorps ou un fragment de celui-ci capable de se lier à la protéine.

7. Procédé selon la revendication 6, dans lequel ledit anticorps ou fragment de celui-ci fait partie d'une trousse.

8. Procédé selon l'une quelconque des revendications précédentes, comprenant en outre (i) collecter des informations de diagnostic ou de pronostic, et (ii) sauvegarder les informations dans un support de données.

9. Utilisation du marqueur CLIC1 en tant que marqueur *in vitro* pour le diagnostic ou le pronostic de carcinome de l'endomètre dans du liquide d'aspiration utérine isolé à partir de l'appareil génital féminin.

10. Utilisation de moyens pour déterminer le niveau du marqueur CLIC 1 dans le procédé selon l'une quelconque des revendications précédentes.
